# EUROPEAN PATENT SPECIFICATION

(11) **EP 1 739 169 B1**
(45) Date of publication and mention of the grant of the patent: **21.10.2009**
(21) Application number: 06013241.2
(22) Date of filing: 27.06.2006
(51) Int. Cl.: C12N 9/96, C12N 9/04, C12Q 1/32

(54) **Clinical diagnostic reagent comprising glucose 6-phosphate dehydrogenase (G6PDH), method for stabilizing G6PDH, and use of a stabilizer for G6PDH**
Klinische diagnostische Reagenz mit Glucose-6-Phosphat-Dehydrogenase (G6PDH), Verfahren zur G6PDH-Stabilisierung und Verwendung eines G6PDH-Stabilisierers
Réactif de diagnostic clinique contenant du glucose-6-phosphate déshydrogénase (G6PDH) et utilisation d'un stabilisateur pour G6PDH

(30) Priority: 29.06.2005 JP 2005189835
(43) Date of publication of application: 03.01.2007
(73) Proprietor: SYSMEX CORPORATION, Kobe-shi, Hyogo 651-0073 (JP)
(72) Inventor: Sakai, Yasuhiro, c/o Sysmex Corporation, Kobe-shi Hyogo 651-0073 (JP)
(74) Representative: HOFFMANN EITLE

(56) References cited:
- WO-A-20/04058958
- WO-A-20/04076656
- US-A- 5 424 204
- STRYER: "Biochemistry" 31 December 1997 (1997-12-31), * page 560 *

## Description

### TECHNICAL FIELD

The present invention relates to a clinical diagnostic reagent comprising glucose 6-phosphate dehydrogenase (G6PDH), a method for stabilizing G6PDH, and a use of a stabilizer for G6PDH.

### BACKGROUND

G6PDH is an enzyme catalyzing a reaction of oxidizing glucose-6-phosphate (referred to hereinafter as G6P) in the presence of a coenzyme such as nicotinamide adenine dinucleotide (NAD) or nicotinamide adenine dinucleotide phosphate (NADP) to form 6-phosphonoglucono-δ-lactone and reduced NAD (NADH) or reduced NADP (NADPH), or its reverse reaction. By utilizing this catalytic action, G6PDH is used in various clinical examination reagents. However, G6PDH in the reagents is unstable and thus a stabilizer for G6PDH should be contained in the reagents.

As a reagent containing G6PDH and the stabilizer described above, there is known a reagent for measurement of the activity of creatine kinase (referred to hereinafter as CK) described in, for example, USP5424204. This reagent contains a hydroxylamine or an aldehyde scavenger as a stabilizer for stabilizing G6PDH.

### SUMMARY

The scope of the present invention is defined solely by the appended claims, and is not affected to any degree by the statements within this summary.

The object of the present invention is to provide a reagent comprising a stabilizer capable of improving the storage stability of G6PDH, as compared with conventionally known stabilizers for G6PDH, as well as a method of stabilizing G6PDH which comprises using the stabilizer to stabilize G6PDH.

A first aspect of the present invention relates to the clinical diagnostic reagent of the present invention comprising G6PDH and a stabilizer for the G6PDH.

A second aspect of the present invention relates to the method for stabilizing G6PDH in a reagent, characterized by allowing a stabilizer to be coexistent with G6PDH in the reagent.

A third aspect of the present invention relates to use of a stabilizer for stabilizing G6PDH.

According to the present invention, there can be provided a reagent for clinical examination excellent in storage stability, which comprises a stabilizer for stabilizing G6PDH. The present invention can also provide a method of stabilizing G6PDH in solution and a use of a stabilizer for G6PDH.

### BRIEF DESCRIPTION OF THE DRAWINGS

Fig. 1 shows a reaction system for measuring an activity of creatine kinase.
Fig. 2 is a graph showing the residual activity (%) of G6PDH in Example 2 and Comparative example 4.
Fig. 3 is a graph showing the residual activity (5) of SH group in Example 2 and Comparative example 4.

### DESCRIPTION OF THE PREFERRED EMBODIMENTS

The reagent that is one embodiment of the present invention comprises glucose-6-phosphate dehydrogenase (G6PDH) and a stabilizer represented by the general formula (I):w 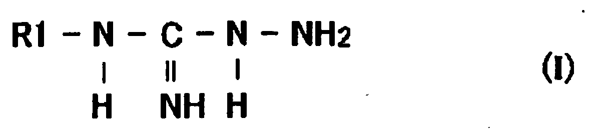 herein R1 represents a hydrogen, a halogen, a hydroxyl group, a substituted or unsubstituted lower alkyl group, a cycloalkyl group, a lower alkoxy group, a lower alkenyl group, a substituted or unsubstituted aralkyl group, a substituted or unsubstituted aryl group, a substituted or unsubstituted heterocyclic group, a lower alkanoyl group, a substituted or unsubstituted aroyl group, a pyridylcarbonyl group, a lower alkoxycarbonyl group, or a substituted or unsubstituted amino group.

The "stabilizer" is a substance which when allowed to be coexistent with G6PDH in a reagent, can inhibit the inactivation of G6PDH and improve the storage stability of G6PDH. In this specification, the "stabilizer" encompasses salts and derivatives of the stabilizer.

As R1 in the stabilizer represented by the general formula (I), the halogen includes fluorine, chlorine, bromine, iodine, astatine, etc.

The alkyl moiety of the lower alkyl group, lower alkoxy group or lower alkoxycarbonyl group includes linear or branched C1 to C6 groups such as a methyl group, ethyl group, propyl group, isopropyl group, butyl group, isobutyl group, sec-butyl group, tert-butyl group, pentyl group, neopentyl group, hexyl group, etc.

The cycloalkyl group includes C3 to C8 groups such as a cyclopropyl group, cyclobutyl group, cyclopentyl group, cyclohexyl group, cycloheptyl group, cyclooctyl group, etc.

The lower alkenyl group includes linear or branched C2 to C6 groups such as a vinyl group, allyl group, isopropenyl group, 4-pentenyl group, 5-hexenyl group, etc.

The aralkyl group includes C7 to C15 groups such as a benzyl group, phenethyl group, benzhydryl group, phenylpropyl group, etc.

The aryl moiety of the aryl group or aroyl group includes a phenyl group, tolyl group, naphthyl group, etc.

The heterocyclic group includes a furyl group, thienyl group, pyrrolyl group, pyranyl group, thiopyranyl group, pyridyl group, thiazolyl group, imidazolinyl group, pyrimidinyl group, triazinyl group, indolyl group, quinolyl group, purinyl group, benzothiazolyl group, etc.

The lower alkanoyl group includes linear or branched C1 to C6 groups such as a formyl group, acetyl group, propionyl group, butyryl group, valeryl group, pivaloyl group, pentanoyl group, etc.

Substituent groups on the lower alkyl group, the number of which is 1 to 3, are the same or different and include, for example, a hydroxy group, lower alkoxy group, carboxy group, lower alkoxycarbonyl group, lower alkylthio group, halogen group, amino group, mono- or dialkyl substituted amino group,phthalimide group,and nitrogen-containing heterocyclic groups such as a pyrrolidinyl group, pyrazolyl group, indolyl group, etc.

The alkyl moiety of the lower alkoxy group, lower alkoxycarbonyl group, lower alkylthio group or alkyl-substituted amino group has the same meaning as defined for the lower alkyl group.

Substituent groups on the aralkyl group, aryl group, aroyl group or heterocyclic group, the number of which is 1 or 5, are the same or different and include, for example, a lower alkyl group, hydroxy group, lower alkoxy group, halogen, nitro group, amino group, etc., and the alkyl moiety of the lower alkyl group or lower alkoxy group has the same meaning as defined for the lower alkyl group.

Substituent groups on the substituted amino group, the number of which is 1 or 2, are the same or different and include, for example, the same lower alkyl group, aralkyl group, substituted or unsubstituted aryl group, lower alkanoyl group, substituted or unsubstituted aroyl group, and alkylidene group as described above, and the alkylidene group includes linear or branched C1 to C6 groups such as a methylene group, ethylidene group, propylidene group, isopropylidene group, butylidene group, isobutylidene group, sec-butylidene group, tert-butylidene group, pentylidene group, isopentylidene group, hexylidene group, etc.

Salts of the stabilizer include, for example, inorganic acid salts such as hydrochloride, hydrobromide, sulfate, nitrate, nitrite, phosphate etc. or organic acid salts such as oxalate, acetate, succinate, fumarate, maleate, tartrate, citrate, carbonate, bicarbonate, etc., or base addition salts such as sodium salt, potassium salt, etc.

In this embodiment, aminoguanidine, aminoguanidine salt or aminoguanidine derivative is preferably used. Aminoguanidine has the structure of the general formula (I) wherein R1 is a hydrogen.

The concentration of the stabilizer in the reagent is not particularly limited insofar as G6PDH can be stabilized. The concentration of G6PDH in the reagent is not particularly limited, but is preferably 10 to 10000 U/L, more preferably 100 to 10000 U/L.

G6PDH may be derived from microorganisms (for example, *Leuconostoc mesenteroides, Bacillus stearothermophiolus, Azotobacter vinelandii, Pseudomonas fluorescens* etc.), yeasts, animal and plants, or may be generated by using genetic recombinant techniques.

The pH of the reagent in solution is preferably 5.0 to 9.0, but is not particularly limited insofar as it is pH unless G6PDH is made unstable. To maintain this pH, a buffer agent is preferably contained in the reagent. As the buffer agent, it is possible to use, for example, Tris-HCl buffer, imidazole-acetate buffer, phosphate buffer, citrate buffer, malate buffer, oxalate buffer,phthalate buffer, glycinebuffer, acetatebuffer, succinate buffer, borate buffer, carbonate buffer, GOOD buffer etc.

In the reagent, the respective ingredients may be in a lyophilized state but are preferably in the form of a solution thereof dissolved in solvent.

The reagent is preferably a reagent kit comprising two reagents, that is, first and second reagents. In this case, the first reagent and/or the second reagent may be in a lyophilized state, but preferably both the reagents are in the form of a solution. G6PDH and the stabilizer may be contained in the first reagent and/or the second reagent.

This reagent is used in clinical examination. By mixing the reagent in this embodiment with a sample, the content or activity of a specific substance in the sample can be measured by monitoring e.g. a change in absorbance. The sample includes, for example, serum, plasma, blood, spinal fluid, urine, semen etc., among which plasma or serum is preferably used.

The substance which can be quantified or measured for its activity by using the reagent in this embodiment is not particularly limited insofar as a G6PDH substrate, glucose-6-phosphate (referred to hereinafter as G6P), is used in the reaction system. The substance which can be quantified includes, for example, neutral fat, inorganic phosphate, lactose, maltose, glycogen, starch, sucrose, glucose, fructose, mannose, creatine phosphate, G6P, fructose-6-phosphate, maltose-6-phosphate, fructose-2-phosphate, L-sorbose-6-phosphate, glucose-1-phosphate, NAD, NADP, uridine triphosphate, adenosine triphosphate (ATP), uridine diphosphate glucose, etc. The substance which can be measured for its activity includes, for example, CK, CKMB, β-galactosidase, α-galactosidase, amyloglucosidase, invertase, transaldolase, galactose-1-phosphate uridyl transferase, phesphoglucose isomerase, fructose diphosphatase, etc.

Hereinafter, the measurement of the activity of CK with the reagent in this embodiment is described in detail.

CK is a dimeric phosphorylase consisting of two subunits. In the CK subunits, there are two types of subunits, that is, B type (brain type) and M type (muscular type). Three kinds of isozymes (CK-MM, CK-MB and CK-BB) occur depending on the combination of the two subunits in CK. CK-MM is contained in a larger amount in the skeletal muscle, CK-MB is contained in a larger amount in the heart muscle, and CK-BB is contained in a larger amount in the brain. CK isozymes occurring in causative sites of diseases such as myocardial infarction and muscular dystrophy are released into blood, and thus the activity of CK isozymes contained in a sample such as serum serves as an important indicator for diagnosis of the above diseases in clinical examination.

Hereinafter, the reagent for measurement of the activity of CK, that is, the reagent according to one embodiment of the present invention, is described in detail.

The reagent for measurement of the activity of CK preferably contains a buffer agent, an SH group-comprising compound (referred to hereinafter SH compound), NAD or NADP, hexokinase (HK) or glucokinase (GK), glucose, adenosine diphosphate (ADP), magnesium ion and creatine phosphate. As the sample subjected to measurement in this case, serum or plasma can be used.

When a sample is mixed with the reagent, CK contained in the sample is activated by the SH compound. CK when released into blood is rapidly inactivated, and thus CK should be activated by the SH compound in order to measure the activity of CK. The SH compound is not particularly limited insofar as CK can be activated, and for example, N-acetyl-L-cysteine (NAC), cysteamine, dithiothreitol (DTT), cysteine, glutathione and thioglycerol can be used. These SH compounds can be used as a mixture of two or more thereof, but are preferably used alone. The concentration of the SH compound in the reagent is 5 to 250 mM, preferably 5 to 70 mM, more preferably 10 to 25 mM.

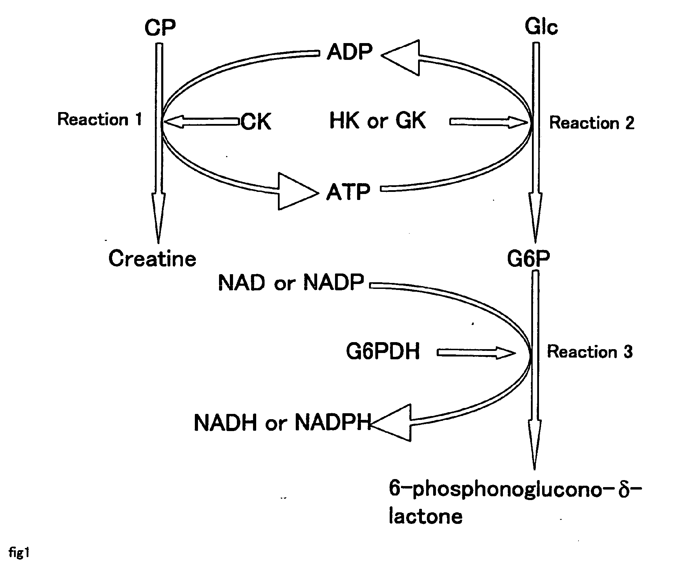

By adding the reagent containing the ingredients described above to the CK-containing sample, the reaction system shown in Fig. 1 is constituted. In Fig. 1, the CK activated by the SH compound catalyzes a reaction of forming creatine and ATP from creatine phosphate (CP) and ADP (Reaction 1). HK or GK contained in the reagent allows G6P and ADP to be formed from glucose (Glc) contained in the reagent and ATP formed in the reaction 1 (Reaction 2). G6PDH contained in the reagent also allows 6-phosphoglucono-δ-lactone and NADH or NADPH to be formed from the G6P formed in the reaction 2 and NAD or NADP (Reaction 3). When NADH or NADPH is formed, the absorbance of a mixed solution of the sample and reagent in the vicinity of 340 nm is increased. By monitoring this increase in absorbance, the activity of CK in the sample can be determined.

The reagent is preferably a reagent kit containing first and second reagents. Preferably, the first reagent contains a buffer agent, G6PDH, the stabilizer, NAD or NADP, hexokinase (HK) or glucokinase (GK), glucose, adenosine diphosphate (ADP), magnesium ion, and SH compound, and the second reagent contains creatine phosphate.

As described above, G6PDH is unstable, and thus a stabilizer for G6PDH can be contained in the reagent. However, any compound conventionally known as the stabilizer deteriorates the SH compound and thus cannot be added in an amount necessary to improve the storage stability of G6PDH, which results in failure to achieve sufficient stabilization of G6PDH. The stabilizer contained in the reagent in this embodiment can be coexistent with G6PDH to improve the storage stability of G6PDH sufficiently without deteriorating the SH compound.

In addition to the ingredients described above, an antiseptic, a chelating agent etc. can be suitably used. As the antiseptic, sodium azide for example can be used. The chelating agent is used to suppress the inhibition of the CK activity by metal ions in the sample, and specifically EDTA, EDDA and salts thereof can be used.

A surface-active compound can also be added to the reagent. For example, a nonionic surfactant, a cationic surfactant, an amphoteric surfactantandasurface-activeproteincanbeused, and specifically Triton, Emulgen series, BSA etc. can be used.

Adenylate kinase is sometimes contained in the sample. Adenylate kinase is contained in a larger amount particularly in a hemolyzed sample and may exert adverse influence on measurement of the activity of CK. To avoid this adverse influence, it is preferable to add an inhibitor for inhibiting the action of adenylate kinase. The type of the inhibitor is not particularly limited insofar as the action of adenylate kinase is inhibited, and for example adenosine monophosphate (AMP) and PIPS diadenosine-5'-pentaphosphate (AP5A) can be used.

By measuring the activity by a double kinetic method, the adverse influence of adenylate kinase can also be avoided. The double kinetic method involves first measuring the activity of adenylate kinase and then adding creatine phosphate to initiate an enzyme reaction with CK to determine the activity of the kinases (total activity of CK and adenylate kinase) contained in the sample. The difference between the two measurement results indicates the activity of CK.

To avoid the adverse influence of adenylate kinase, a sample containing the above-mentioned inhibitor is preferably used to measure the activity by the double kinetic method.

By incorporating an antibody specifically recognizing the M type subunit of CK (referred to hereinafter as anti-CK-M antibody) into the above-described reagent for measurement of the activity of CK, the activity of CK-MB in a sample can be measured (Wurzburg et al. , 1977, J. Clin. Chem. Clin. Biochem., 15:131-135). The anti-CK-M antibody may be a polyclonal antibody or monoclonal antibody insofar as it is an antibody specifically recognizing the M type subunit, or a mixture thereof may also be used. An antibody fragment and a derivative thereof can also be used. Examples of the antibody fragment and a derivative thereof include Fab, Fab', F(ab)₂ and sFv fragment (Blazar et al., 1997, J. Immunol., 159:5821-5833 and Bird et al., 1988, Science, 242:423-426). The antibody subclass is not limited to IgG and may be IgM or the like.

### EXAMPLE 1

### <Preparation of reagent>

Compounds shown below were mixed to prepare a reagent for measurement of CK activity. The shown concentration of each ingredient is the concentration thereof in the reagent.

| | |
|---|---|
| Imidazole | 125 mM |
| EDTA | 2.5 mM |
| Magnesium acetate | 12.5 mM |
| ADP | 2.5 mM |
| AMP | 6.25 mM |
| AP5A | 12.5 µM |
| Glucose | 25 mM |
| NADP | 2.5 mM |
| NAC | 25 mM |
| G6PDH | 1875 U/L |
| Hexokinase | 3750 U/L |

The pH of the reagent was adjusted to 6.6.

The reagent for measurement of CK activity consists of the above reagent (first reagent) and the creatine phosphate-containing reagent (second reagent), but in this example, the reagent is subjected to heat loading analysis in order to analyze the stability of G6PDH and NAC, and thus the second reagent is not used.

Aminoguanidine was added to the above composition to prepare three kinds of reagents different in the concentration of aminoguanidine. The concentrations of aminoguanidine in these reagents were 10 mM, 20 mM and 40 mM respectively. After these reagents were subjected to heat loading at 37°C for 5 days, the residual activity of G6PDH was measured and the residual amount of SH group in NAC was quantified.

The activity of G6PDH was measured in the following manner. First, a solution wherein 20 mM magnesium chloride, 0.6 mM NADP and 0.5 mM G6P were dissolved in a solution containing 0.1 M glycylglycine (pH 8.5) was prepared. When 300 µl of this solution was mixed with 1 µl of the first reagent, NADPH is formed by the activity of G6DPH. When NADPH is formed, the absorbance at a wavelength of 340 nm is increased, and thus this increase is measured to determine the activity of G6PDH.

The quantification of SH group was carried out by using DTNB (5,5'-dithiobis(2-nitrobenzoic acid)). When DTNB is added to the reagent where NAC is present, disulfide linkages corresponding to the amount of SH groups in the NAC are cleaved to generate 5-mercapto-2-nitrobenzoic acid. When 5-mercapto-2-nitrobenzoic acid is generated, the absorbance at a wavelength of 412 nm is increased, and thus this increase can be measured to determine the amount of SH groups in the reagent.

### COMPARATIVE EXAMPLES 1 to 3

In Comparative Example 1, measurement of the residual activity of G6PDH and quantification of the residual amount of SH group of NAC were carried out in the same manner as in Example 1 except that not aminoguanidine but acetohydrazine known as G6PDH stabilizer was added to the composition described above, to prepare 2 kinds of reagents different in acetohydrazine concentration. In the respective reagents, the acetohydrazine concentrations were 20 mM and 40 mM respectively.

In Comparative Example 2, measurement of the residual activity of G6PDH and quantification of the residual amount of SH group of NAC were carried out in the same manner as in Example 1 except that not aminoguanidine but hydrazine known as G6PDH stabilizer was added to the composition described above, to prepare 2 kinds of reagents different in hydrazine concentration. In the respective reagents, the hydrazine concentrations were 20 mM and 40 mM respectively.

In Comparative Example 3, measurement of the residual activity of G6PDH and quantification of the residual amount of SH group of NAC were carried out without adding any stabilizer.

The measurement results in Example 1 and Comparative Examples 1 to 3 are shown in Table 1 below. In Table 1, the residual activity of G6PDH and the residual amount of SH group of NAC are expressed in percentage. These values are those relative to the activity of G6PDH and the amount of SH group of NAC in each reagent which without subjection to heat loading, was measured by the method described in Example 1.

**Table 1**

| stabilizer | | concentration (mM) | G6PDH residual activity (%) | SH group residual amount (%) |
|---|---|---|---|---|
| aminoguanidine | Example1 | 10 | 88.2 | 92.2 |
| | | 20 | 92.6 | 95.4 |
| | | 40 | 94.8 | 94.1 |
| acetohydrazine | Comparative example1 | 20 | 84.3 | 78.4 |
| | | 40 | 87.8 | 77.7 |
| hydrazine | Comparative example2 | 20 | 87.8 | 54.7 |
| | | 40 | 91.9 | 56.2 |
| no stabilizer | Comparative example3 | 0 | 63.2 | 95.2 |

In Comparative Example 1, it was revealed that by heat loading, the activity of G6PDH was lost, and NAC was deteriorated to reduce the amount of SH group.

In Comparative Example 2, the reduction of G6PDH activity was suppressed to a certain degree, but NAC was deteriorated thus significantly reducing the amount of SH group.

In Comparative Example 3, NAC was not deteriorated and thus the amount of SH group was not reduced, but because no stabilizer was added, the activity of G6PDH was lost.

In Example 1, the activity of G6PDH could be maintained without reducing the amount of SH group.

From the foregoing, it was revealed that when aminoguanidine is added as stabilizer to the reagent, the storage stability of G6PDH can be improved without deteriorating NAC.

### EXAMPLE 2

Aminoguanidine was added at a concentration of 20 mM to the reagent in Example 1 and subjected to heat loading at 37°C for 30 days, followed by measuring the activity of G6PDH and quantifying the SH group of NAC. Before the heat loading and 7 days, 14 days, 20 days and 30 days after initiation of the heat loading, the activity measurement and quantification were carried out in the same manner as in Example 1.

### COMPARATIVE EXAPMLE 4

Firstreagents (Reagents A, B, C, D, EandF) for CK activity measurement available from A, B, C, D, E and F companies respectively were subjected to heat loading at 37°C, followed by measuring the activity of G6PDH and quantifying the SH group of NAC. This activity measurement and quantification were carried out in the same manner as in Example 2.

As a control, the reagent prepared in Example 1 (the aminoguanidine-free control reagent (control reagent)) was subjected to heat loading at 37°C followed by measuring the activity of G6PDH and quantifying the SH group of NAC. This activity measurement and quantification were carried out in the same manner as in Example 2.

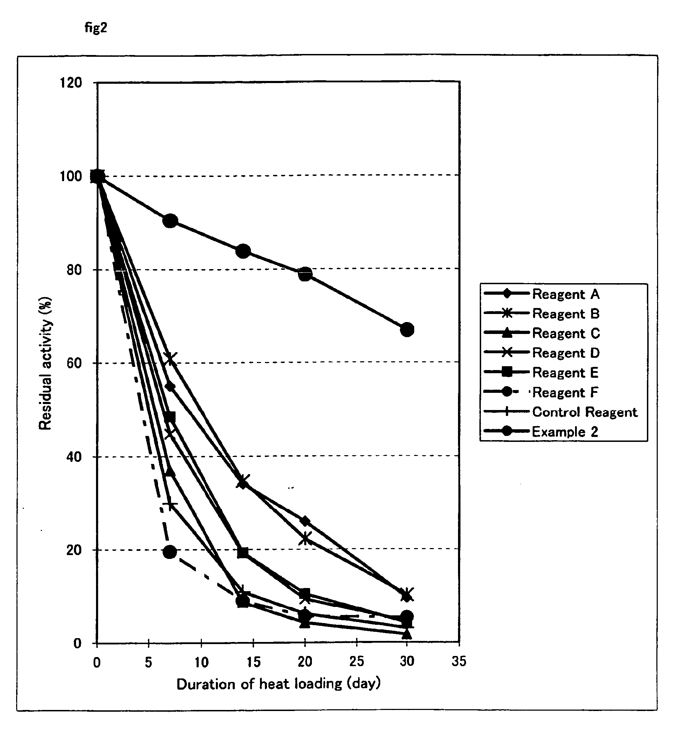

The results of measurement of the activity of G6PDH in Example 2 and Comparative Example 4 are shown in Table 2 and Fig. 2. In Fig. 2, the residual activity (%) of G6PDH is shown on the ordinate and the period of heat loading (number of days) is shown on the abscissa.

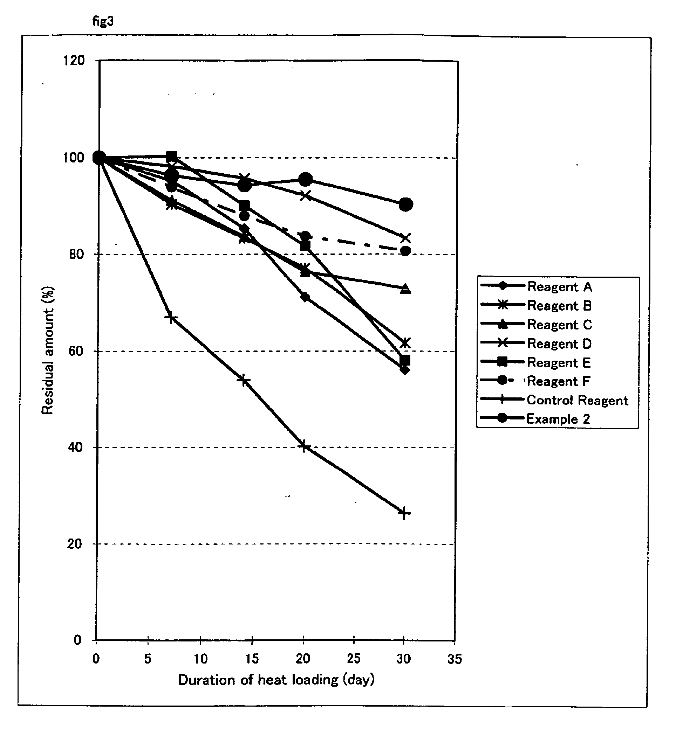

The results of quantifying the SH group in Example 2 and Comparative Example 4 are shown in Table 3 and Fig. 3. In Fig. 3, the residual amount (%) of SH group is shown on the ordinate and the period of heat loading (number of days) is shown on the abscissa.

**[Table 2]**

| Residual acthrity of G6PDH (%) | | | | | | | | |
|---|---|---|---|---|---|---|---|---|
| | Reagent A | Reagent B | Reagent C | Reagent D | Reagent E | Reagent F | Control Reagent | Example 2 |
| Before heat loading | 100.0 | 100.0 | 100.0 | 100.0 | 100.0 | 100.0 | 100.0 | 100.0 |
| 7days after the initiation of heat loading | 55.0 | 60.9 | 37.0 | 44.8 | 48.5 | 19.5 | 29.9 | 90.5 |
| 14days after the initiation of heat loading | 34.1 | 34.7 | 8.7 | 19.3 | 19.3 | 9.1 | 11.0 | 83.9 |
| 20days after the initiation of heat loading | 26.0 | 22.2 | 4.3 | 9.4 | 10.5 | 5.7 | 6.2 | 78.9 |
| 30days after the initiation of heat loading | 9.6 | 10.3 | 1.7 | 4.6 | 4.2 | 5.4 | 3.1 | 66.9 |

**[Table 3]**

| Residual amount of SH group (%) | | | | | | | | |
|---|---|---|---|---|---|---|---|---|
| | Reagent A | Reagent B | Reagent C | Reagent D | Reagent E | Reagent F | Control Reagent | Example 2 |
| Before heat loading | 100.0 | 100.0 | 100.0 | 100.0 | 100.0 | 100.0 | 100.0 | 100.0 |
| 7days after the initiation of heat loading | 95.3 | 90.4 | 91.2 | 98.3 | 100.3 | 93.9 | 67.0 | 96.4 |
| 14days after the initiation of heat loading | 85.3 | 83.3 | 83.7 | 95.8 | 90.1 | 88.0 | 53.9 | 94.3 |
| 20days after the initiation of heat loading | 71.1 | 77.1 | 76.4 | 92.2 | 81.7 | 83.8 | 40.2 | 95.5 |
| 30days after the initiation of heat loading | 56.1 | 61.7 | 72.9 | 83.3 | 58.0 | 80.7 | 26.3 | 90.3 |

As can be seen from Table 2 and Fig. 2, any of the commercial reagents A to F and the control reagent, upon heat loading, caused a significant reduction in G6PDH activity, while the reagent in Example 2 to which aminoguanidine was added showed 66.9% residual activity even after heat loading for 30 days. As can be seen from Table 3 and Fig. 3, the reagent in Example 2 had a larger amount of remaining SH groups than in the reagents A to F and the control reagent.

From the foregoing, it was found that the reagent to which aminoguanidine was added as a stabilizer provides higher storage stability for G6PDH and NAC than the aminoguanidine-free reagent and the commercial reagents.

**table 1**

| stabilizer | | concentration (mM) | G6PDH residual activity (%) | SH group residual amount (%) |
|---|---|---|---|---|
| aminoguanidine | Example 1 | 10 | 88,2 | 92,2 |
| | | 20 | 92,6 | 95,4 |
| | | 40 | 94,8 | 94,1 |
| acetohydrazine | Comparative example 1 | 20 | 84,3 | 78,4 |
| | | 40 | 87,8 | 77,7 |
| hydrazine | Comparative example2 | 20 | 87,8 | 54,7 |
| | | 40 | 91,9 | 56,2 |
| no stabilizer | Comparative example3 | 0 | 63,2 | 95,2 |

**table2 Residual activity of G6PDH (%)**

| | Reagent A | Reagent B | Reagent C | Reagent D | Reagent E | Reagent F | Control Reagent | Example 2 |
|---|---|---|---|---|---|---|---|---|
| Before heat loading | 100,0 | 100,0 | 100,0 | 100,0 | 100,0 | 100,0 | 100,0 | 100,0 |
| 7days after the initiation of heat loading | 55,0 | 60,9 | 37,0 | 44,8 | 48,5 | 19,5 | 29.9 | 90,5 |
| 14drays after the initiation of heat loading | 34,1 | 34,7 | 8,7 | 19,3 | 19,3 | 9,1 | 11,0 | 83,9 |
| 20days after the initiation of heat loading | 26,0 | 22,2 | 4,3 | 9,4 | 10,5 | 5,7 | 6,2 | 78,9 |
| 30days after the initiation of heat loading | 9,6 | 10,3 | 1,7 | 4,6 | 4.2 | 5,4 | 3,1 | 66,9 |

**table3**

| Residual amount of SH group (%) | | | | | | | | |
|---|---|---|---|---|---|---|---|---|
| | Reagent A | Reagent B | Reagent C | Reagent D | Reagent E | Reagent F | Control Reagent | Example 2 |
| Before heat loading | 100,0 | 100,0 | 100,0 | 100,0 | 100,0 | 100,0 | 100,0 | 100,0 |
| 7days after the initiation of heat loading | 95,3 | 90,4 | 91,2 | 98,3 | 100,3 | 93,9 | 67,0 | 96,4 |
| 14drays after the initiation of heat loading | 85,3 | 83,3 | 83,7 | 95,8 | 90,1 | 88,0 | 53,9 | 94,3 |
| 20days after the initiation of heat loading | 71,1 | 77,1 | 76,4 | 92,2 | 81,7 | 83,8 | 40,2 | 95,5 |
| 30days after the initiation of heat loading | 56,1 | 61,7 | 72.9 | 83,3 | 58,0 | 80,7 | 26,3 | 90,3 |

## Claims

1. A clinical diagnostic reagent, comprising glucose 6-phosphate dehydrogenase (G6PDH) and a stabilizer for the G6PDH represented by formula (I): 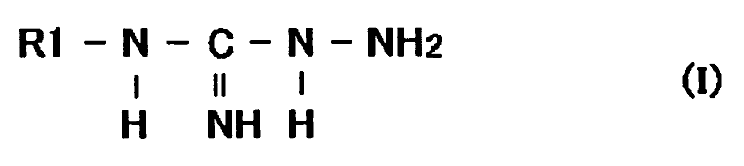 wherein R1 represents a hydrogen, a halogen, a hydroxyl group, a substituted or unsubstituted lower alkyl group, a cycloalkyl group, a lower alkoxy group, a lower alkenyl group, a substituted or unsubstituted aralkyl group, a substituted or unsubstituted aryl group, a substituted or unsubstituted heterocyclic group, a lower alkanoyl group, a substituted or unsubstituted aroyl group, a pyridylcarbonyl group, a lower alkoxycarbonyl group, or a substituted or unsubstituted amino group.

2. The reagent according to claim 1, wherein R1 represents a hydrogen, a halogen, a hydroxyl group, a substituted or unsubstituted C1 to C6 alkyl group, a cycloalkyl group, a C1 to C6 alkoxy group, a C2 to C6 alkenyl group, a substituted or unsubstituted aralkyl group, a substituted or unsubstituted aryl group, a substituted or unsubstituted heterocyclic group, a C1 to C6 alkanoyl group, a substituted or unsubstituted aroyl group, a pyridylcarbonyl group, a C1 to C6 alkoxycarbonyl group, or a substituted or unsubstituted amino group.

3. The reagent according to claim 1 or 2, wherein the R1 is a hydrogen.

4. The reagent according to any of claims 1 to 3, further comprising at least one compound selected from the group consisting of a coenzyme corresponding to the G6PDH, a glucokinase, a hexokinase, a glucose, an adenosine diphosphate, a magnesium ion, a SH compound containing a thiol group, and a creatine phosphate.

5. The reagent according to claim 4, wherein the coenzyme is selected from the group consisting of nicotinamide adenine dinucleotide and nicotinamide adenine dinucleotide phosphate.

6. The reagent according to claim 4 or 5, wherein the kinase is selected from the group consisting of hexokinase and glucokinase.

7. The reagent according to any of claims 4 to 6, wherein the SH compound is selected from the group consisting of N-acetyl-L-cysteine (NAC), cysteamine, dithiothreitol (DTT), cysteine, glutathione and thioglycerol.

8. The reagent according to any of claims 1 to 7, wherein the reagent is used for the measurement of activity of creatine kinase in a sample, and further comprises a SH compound containing a thiol group, a hexokinase (HK) or a glucokinase (GK), a glucose, an adenosine diphosphate (ADP), and a creatine phosphate.

9. A method for stabilizing glucose 6-phosphate dehydrogenase (G6PDH) in a clinical diagnostic reagent, **characterized by** adding a stabilizer to the clinical diagnostic reagent, wherein the stabilizer is represented by formula (I): 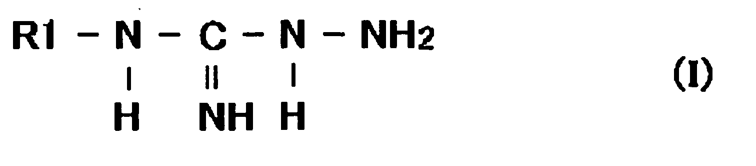 wherein R1 represents a hydrogen, a halogen, a hydroxyl group, a substituted or unsubstituted lower alkyl group, a cycloalkyl group, a lower alkoxy group, a lower alkenyl group, a substituted or unsubstituted aralkyl group, a substituted or unsubstituted aryl group, a substituted or unsubstituted heterocyclic group, a lower alkanoyl group, a substituted or unsubstituted aroyl group, a pyridylcarbonyl group, a lower alkoxycarbonyl group, or a substituted or unsubstituted amino group.

10. The method according to claim 9, wherein the reagent comprises a SH compound containing a thiol group, and the stabilizer further stabilizes the SH compound.

11. Use of a stabilizer for stabilizing glucose 6-phosphate dehydrogenase (G6PDH), wherein the stabilizer is represented by formula (I): 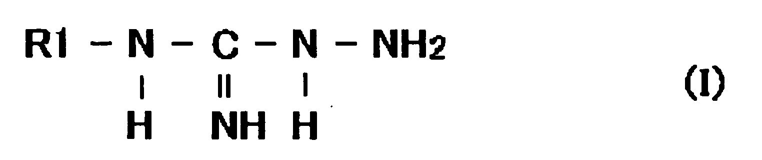 wherein R1 represents a hydrogen, a halogen, a hydroxyl group, a substituted or unsubstituted lower alkyl group, a cycloalkyl group, a lower alkoxy group, a lower alkenyl group, a substituted or unsubstituted aralkyl group, a substituted or unsubstituted aryl group, a substituted or unsubstituted heterocyclic group, a lower alkanoyl group, a substituted or unsubstituted aroyl group, a pyridylcarbonyl group, a lower alkoxycarbonyl group, or a substituted or unsubstituted amino group.

## Patentansprüche

1. Klinisches Diagnostikreagens, umfassend Glucose-6-Phosphat-Dehydrogenase (G6PDH) und einen Stabilisator für die G6PDH, dargestellt durch Formel (I): 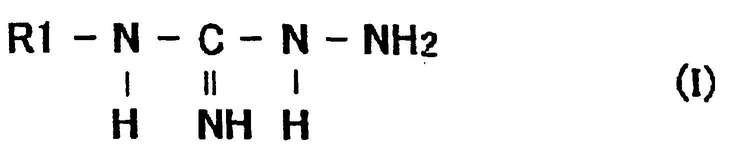 worin R1 ein Wasserstoff, ein Halogen, eine Hydroxylgruppe, eine substituierte oder unsubstituierte Niederalkylgruppe, eine Cycloalkylgruppe, eine Niederalkoxygruppe, eine Niederalkenylgruppe, eine substituierte oder unsubstituierte Aralkylgruppe, eine substituierte oder unsubstituierte Arylgruppe, eine substituierte oder unsubstituierte heterocyclische Gruppe, eine Niederalkanoylgruppe, eine substituierte oder unsubstituierte Aroylgruppe, eine Pyridylcarbonylgruppe, eine Niederalkoxycarbonylgruppe oder eine substituierte oder unsubstituierte Aminogruppe darstellt.

2. Reagens gemäß Anspruch 1, worin R1 ein Wasserstoff, ein Halogen, eine Hydroxylgruppe, eine substituierte oder unsubstituierte C1-C6-Alkylgruppe, eine Cycloalkylgruppe, eine C1-C6-Alkoxygruppe, eine C2-C6-Alkenylgruppe, eine substituierte oder unsubstituierte Aralkylgruppe, eine substituierte oder unsubstituierte Arylgruppe, eine substituierte oder unsubstituierte heterocyclische Gruppe, eine Cl-C6-Alkanoylgruppe, eine substituierte oder unsubstituierte Aroylgruppe, eine Pyridylcarbonylgruppe, eine C1-C6-Alkoxycarbonylgruppe oder eine substituierte oder unsubstituierte Aminogruppe darstellt.

3. Reagens gemäß Anspruch 1 oder 2, worin das R1 ein Wasserstoff ist.

4. Reagens gemäß einem der Ansprüche 1 bis 3, ferner umfassend wenigstens eine Verbindung, die ausgewählt ist aus der Gruppe, bestehend aus einem dem G6PDH entsprechenden Coenzym, einer Glucokinase, einer Hexokinase, einer Glucose, einem Adenosindiphosphat, einem Magnesiumion, einer SH-Verbindung, die eine Thiolgruppe enthält, und einem Creatinphosphat.

5. Reagens gemäß Anspruch 4, worin das Coenzym ausgewählt ist aus der Gruppe, bestehend aus Nicotinamid-Adenin-Dinucleotid und Nicotinamid-Adenin-Dinucleotid-Phosphat.

6. Reagens gemäß Anspruch 4 oder 5, worin die Kinase ausgewählt ist aus der Gruppe, bestehend aus Hexokinase und Glucokinase.

7. Reagens gemäß einem der Ansprüche 4 bis 6, worin die SH-Verbindung ausgewählt ist aus der Gruppe, bestehend aus N-Acetyl-L-cystein (NAC), Cysteamin, Dithiothreitol (DTT), Cystein, Glutathion und Thioglycerol.

8. Reagens gemäß einem der Ansprüche 1 bis 7, worin das Reagens zur Messung der Aktivität der Creatinkinase in einer Probe verwendet wird und ferner eine SH-Verbindung, die eine Thiolgruppe enthält, eine Hexokinase (HK) oder eine Glucokinase (GK), eine Glucose, ein Adenosin-Diphosphat (ADP) und ein CreatinPhosphat umfasst.

9. Verfahren zur Stabilisierung von Glucose-6-phosphat-Dehydrogenase (G6PDH) in einem klinischen Diagnostikreagens, **gekennzeichnet durch** Zugabe eines Stabilisators zu dem klinischen Diagnostikreagens, worin der Stabilisator **durch** Formel (I) dargestellt ist: 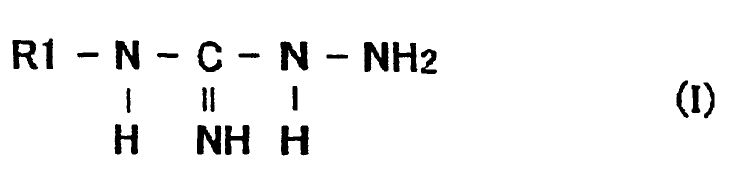 worin R1 ein Wasserstoff, ein Halogen, eine Hydroxylgruppe, eine substituierte oder unsubstituierte Niederalkylgruppe, eine Cycloalkylgruppe, eine Niederalkoxygruppe, eine Niederalkenylgruppe, eine substituierte oder unsubstituierte Aralkylgruppe, eine substituierte oder unsubstituierte Arylgruppe, eine substituierte oder unsubstituierte heterocyclische Gruppe, eine Niederalkanoylgruppe, eine substituierte oder unsubstituierte Aroylgruppe, eine Pyridylcarbonylgruppe, eine Niederalkoxycarbonylgruppe oder eine substituierte oder unsubstituierte Aminogruppe darstellt.

10. Verfahren gemäß Anspruch 9, worin das Reagens eine SH-Verbindung, die eine Thiolgruppe enthält, umfasst und der Stabilisator ferner die SH-Verbindung stabilisiert.

11. Verwendung eines Stabilisators zum Stabilisieren von Glucose-6-phosphat-Dehydrogenase (G6PDH), worin der Stabilisator durch Formel (I) dargestellt ist: 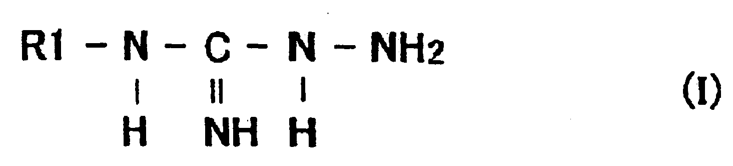 worin R1 ein Wasserstoff, ein Halogen, eine Hydroxylgruppe, eine substituierte oder unsubstituierte Niederalkylgruppe, eine Cycloalkylgruppe, eine Niederalkoxygruppe, eine Niederalkenylgruppe, eine substituierte oder unsubstituierte Aralkylgruppe, eine substituierte oder unsubstituierte Arylgruppe, eine substituierte oder unsubstituierte heterocyclische Gruppe, eine Niederalkanoylgruppe, eine substituierte oder unsubstituierte Aroylgruppe, eine Pyridylcarbonylgruppe, eine Niederalkoxycarbonylgruppe oder eine substituierte oder unsubstituierte Aminogruppe darstellt.

## Revendications

1. Réactif de diagnostic clinique, comprenant de la glucose-6-phosphate déshydrogénase (G6PDH) et un stabilisant de la G6PDH représenté par la formule (I) : 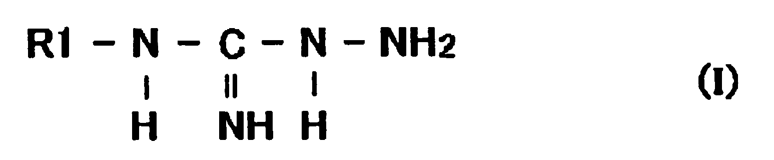 dans laquelle R1 représente un hydrogène, un halogène, un groupe hydroxyle, un groupe alkyle inférieur substitué ou non substitué, un groupe cycloalkyle, un groupe alcoxy inférieur, un groupe alcényle inférieur, un groupe aralkyle substitué ou non substitué, un groupe aryle substitué ou non substitué, un groupe hétérocyclique substitué ou non substitué, un groupe alcanoyle inférieur, un groupe aroyle substitué ou non substitué, un groupe pyridylcarbonyle, un groupe alcoxycarbonyle inférieur, ou un groupe amino substitué ou non substitué.

2. Réactif selon la revendication 1, dans lequel R1 représente un hydrogène, un halogène, un groupe hydroxyle, un groupe alkyle en C1 à C6 substitué ou non substitué, un groupe cycloalkyle, un groupe alcoxy en C1 à C6, un groupe alcényle en C2 à C6, un groupe aralkyle substitué ou non substitué, un groupe aryle substitué ou non substitué, un groupe hétérocyclique substitué ou non substitué, un groupe alcanoyle en C1 à C6, un groupe aroyle substitué ou non substitué, un groupe pyridylcarbonyle, un groupe alcoxycarbonyle en C1 à C6, ou un groupe amino substitué ou non substitué.

3. Réactif selon la revendication 1 ou 2, dans lequel le R1 est un hydrogène.

4. Réactif selon l'une quelconque des revendications 1 à 3, comprenant en outre au moins un composé sélectionné dans le groupe constitué par un coenzyme correspondant à la G6PDH, une glucokinase, une hexokinase, un glucose, une adénosine diphosphate, un ion magnésium, un composé de type SH contenant un groupe thiol, et une créatine phosphate.

5. Réactif selon la revendication 4, dans lequel le coenzyme est sélectionné dans le groupe constitué par le nicotinamide-adénine-dinucléotide et le nicotinamide-adénine-dinucléotide-phosphate.

6. Réactif selon la revendication 4 ou 5, dans lequel la kinase est sélectionnée dans le groupe constitué par l'hexokinase et la glucokinase.

7. Réactif selon l'une quelconque des revendications 4 à 6, dans lequel le composé de type SH est sélectionné dans le groupe constitué par la N-acétyl-L-cystéine (NAC), la cystéamine, le dithiothréitol (DTT), la cystéine, le glutathion et le thioglycérol.

8. Réactif selon l'une quelconque des revendications 1 à 7, ledit réactif étant utilisé pour la mesure d'activité de la créatine kinase dans un échantillon, et comprenant en outre un composé de type SH contenant un groupe thiol, une hexokinase (HK) ou une glucokinase (GK), un glucose, une adénosine diphosphate (ADP), et une créatine phosphate.

9. Procédé pour stabiliser de la glucose-6-phosphate déshydrogénase (G6PDH) dans un réactif de diagnostic clinique, **caractérisé par** l'addition d'un stabilisant au réactif de diagnostic clinique, dans lequel le stabilisant est représenté par la formule (I) : 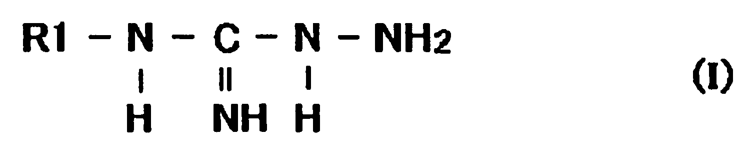 dans laquelle R1 représente un hydrogène, un halogène, un groupe hydroxyle, un groupe alkyle inférieur substitué ou non substitué, un groupe cycloalkyle, un groupe alcoxy inférieur, un groupe alcényle inférieur, un groupe aralkyle substitué ou non substitué, un groupe aryle substitué ou non substitué, un groupe hétérocyclique substitué ou non substitué, un groupe alcanoyle inférieur, un groupe aroyle substitué ou non substitué, un groupe pyridylcarbonyle, un groupe alcoxycarbonyle inférieur, ou un groupe amino substitué ou non substitué.

10. Procédé selon la revendication 9, dans lequel le réactif comprend un composé de type SH contenant un groupe thiol, et le stabilisant stabilise en outre le composé de type SH.

11. Utilisation d'un stabilisant pour stabiliser de la glucose-6-phosphate déshydrogénase (G6PDH), dans laquelle le stabilisant est représenté par la formule (I) : 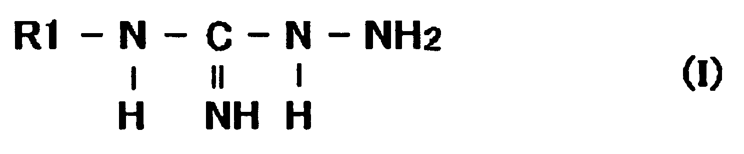 dans laquelle R1 représente un hydrogène, un halogène, un groupe hydroxyle, un groupe alkyle inférieur substitué ou non substitué, un groupe cycloalkyle, un groupe alcoxy inférieur, un groupe alcényle inférieur, un groupe aralkyle substitué ou non substitué, un groupe aryle substitué ou non substitué, un groupe hétérocyclique substitué ou non substitué, un groupe alcanoyle inférieur, un groupe aroyle substitué ou non substitué, un groupe pyridylcarbonyle, un groupe alcoxycarbonyle inférieur, ou un groupe amino substitué ou non substitué.
